# EUROPEAN PATENT SPECIFICATION

(11) **EP 2 118 310 B1**
(45) Date of publication and mention of the grant of the patent: **06.03.2013**
(21) Application number: 07871745.1
(22) Date of filing: 28.12.2007
(51) Int. Cl.: C12Q 1/68

(54) **SYSTEMS AND METHODS FOR DETECTING NUCLEIC ACIDS**
SYSTEME UND VERFAHREN ZUM NACHWEIS VON NUKLEINSÄUREN
SYSTEMES ET PROCEDES POUR LA DETECTION D'ACIDE NUCLEIQUE

(30) Priority: 16.01.2007 US 880428 P; 18.01.2007 US 880964 P; 29.12.2006 US 877610 P
(43) Date of publication of application: 18.11.2009
(73) Proprietor: Applied Biosystems, LLC, Foster City, CA 94404-1128 (US)
(72) Inventor: SCABOO, Kristian, Castro Valley, CA 94546 (US); AIVAZACHVILI, Vissarion, Oakland, CA 94618 (US); LIU, Timothy, Fremont, CA 94555 (US); EASON, Robert, Los Gatos, CA 95033 (US); FAULSTICH, Konrad, Fremont, CA 94555 (US)
(74) Representative: Wöhler, Christian
(86) International application number: PCT/US2007/089010
(87) International publication number: WO 2008/083261

(56) References cited:
- WO-A-01/06016
- WO-A-2005/010199
- WO-A-2005/068660
- WO-A-2007/011946
- US-A- 5 487 972
- US-A- 5 843 669
- US-A1- 2005 255 512
- US-A1- 2006 246 475
- ALLAWI HATIM T ET AL: "Invader plus method detects herpes simplex virus in cerebrospinal fluid and simultaneously differentiates types 1 and 2." JOURNAL OF CLINICAL MICROBIOLOGY SEP 2006, vol. 44, no. 9, September 2006 (2006-09), pages 3443-3447, XP002480721 ISSN: 0095-1137

### SEQUENCE LISTING

<110> SCABCO, Kristian M AI VAZACHVI LI, Vissarion LI U, Timothy EASCN, Robert G FAULSTICH, Konrad
<120> METHODS AND SYSTEMS FOR DETECTING NUCLEIC ACIDS
<130> 70043.0045WOU1
<150> 60/877,610
   <151> 2006-12-29
<150> 60/880,428
   <151> 2007-01-16
<150> 60/880,964
   <151> 2007-01-18
<160> 15
<170> Patent Inversion 3. 4
<210> 1
   <211> 40
   <212> DNA
   <213> Artificial Sequence
<220>
   <223> chemically- synthesi zed capt ur e pr obe
<400> 1
   aaaaaaaccc cagcaat t ca agt gt gt t ga gagct t t gat 40
<210> 2
   <211> 25
   <212> DNA
   <213> Artificial Sequence
<220>
   <223> chemically-synthesized capture pr obe
<400> 2
   aaaaaat t ga gagctttgat t cgt g 25
<210> 3
   <211> 37
   <212> DNA
   <213> Artificial Sequence
<220>
   <223> chem cal I y-synthesized PCR hybridization pr obe
<400> 3
   atcaaagctc tcaacgcctg caagt cct aa gacgcca 37
<210> 4
   <211> 45
   <212> DNA
   <213> Artificial Sequence
<220>
   <223> chemically-synthesized hybridization pr obe
<400> 4
   gt t act t cgt t cgat t gt ct ggact t at aa t gct gaact t ct ggt 45
<210> 5
   <211> 41
   <212> DNA
   <213> Artificial Sequence
<220>
   <223> chemically-synthesized hybridization pr obe
<400> 5
   ct t cgt t cga t t gt ct ggac t t at aat gct gaact t ct gg t 41
<210> 6
   <211> 39
   <212> DNA
   <213> Artificial Sequence
<220>
   <223> chemically-synthesized hybridization probe
<400> 6
   tcgttcgat t gt ct ggact t at aat gct ga act t ct ggt 39
<210> 7
   <211> 19
   <212> DNA
   <213> Artificial Sequence
<220>
   <223> chemically-synthesized PCR primer
<400> 7
   catggcacca ccagcatct 19
<210> 8
   <211> 20
   <212> DNA
   <213> Artificial Sequence
<220>
   <223> chemically-synthesized PCR primer
<400> 8
   at ccgcgt gt t ctttt t cga 20
<210> 9
   <211> 42
   <212> DNA
   <213> Artificial Sequence
<220>
   <223> chemically-synthesized cl eaved tag
<400> 9
   cacgaatcaa agctctcaac gcctgcaagt cctaagacgc ca 42
<210> 10
   <211> 45
   <212> DNA
   <213> Artificial Sequence
<220>
   <223> chemically-synthesized capt ur e probe
<400> 10
   gt gct t agt t tcgagagttg tgtgaactta acgaccccaa aaaaa 45
<210> 11
   <211> 42
   <212> DNA
   <213> Artificial Sequence
<220>
   <223> chemically-synthesized cleaved t ag
<400> 11
   cacgaatcaa agctctcaac gcctgcaagt cctaagacgc ca 42
<210> 12
   <211> 25
   <212> DNA
   <213> Artificial Sequence
<220>
   <223> chemically-synthesized capt ur e probe
<400> 12
   aaaaaagt gc t t agt t t cga gagt t 25
<210> 13
   <211> 37
   <212> DNA
   <213> Artificial Sequence
<220>
   <223> chemically-synthesized cleaved t ag
<400> 13
   at caaagct c t caacgcct g caagt cct aa gacgcca 37
<210> 14
   <211> 25
   <212> DNA
   <213> Artificial Sequence
<220>
   <223> chemically-synthesized capt ur e probe
<400> 14
   aaaaaagt gc t t agt t t cga gagt t 25
<210> 15
   <211> 23
   <212> DNA
   <213> Artificial Sequence
<220>
   <223> chemically-synthesized target-specific segment
<400> 15
   cgcctgcaag tcctaagacg cca 23

## Description

*The section headings used herein are for organizational purposes only and should not be construed as limiting the subject matter described herein in any way.*

### Field

This application relates generally to systems and methods for detecting biological molecules and, in particular, to systems and methods for detecting nucleic acids in a sample.

### Introduction

Nucleic acid amplification may be performed in conjunction with a variety of assays. Such assays may be qualitative, for example when used to evaluate a biological sample. However, a wide variety of biological applications could be improved by the ability to detect the amplification of target nucleic acids, without requiring either cumbersome blotting techniques, or the expensive and delicate equipment typically required for optical methods.

Accordingly, there still exists a need for improved methods for detecting nucleic acids in a sample.

### SUMMARY

According to a first embodiment, a method of detecting a target nucleic acid in a sample is provided which comprises:
(a) incubating the sample with:
   (1) a primer which hybridizes to at least a portion of the target nucleic acid;
   (2) a hybridization probe comprising first and second regions, wherein the first region hybridizes to at least a portion of the target nucleic acid and the second region does not hybridize to the target nucleic acid, the second region comprising a detectable label; and
   (3) a polymerase and an enzyme comprising an nuclease activity, wherein the polymerase extends the hybridized primer in the direction of the hybridized probe and the nuclease activity of the enzyme cleaves the hybridized probe to thereby release a probe fragment comprising the second region and the detectable label;
(b) allowing the primer and the hybridization probe to hybridize to target nucleic acid in the sample;
(c) allowing the polymerase to extend the hybridized primer;
(d) allowing the nuclease activity of the enzyme to cleave the hybridized hybridization probe to thereby release the probe fragment;
(e) contacting the sample with a surface of a solid support, wherein the surface of the solid support comprises one or more capture probes each of which hybridizes to at least a portion of the second region of the probe fragment;
(f) allowing the capture probes to hybridize to probe fragment in the sample to form a probe fragment/capture probe complex; and
(g) detecting the label on the surface of the solid support,
wherein the hybridization probe is substantially single stranded at the Tm of the probe fragment/capture probe complex, and wherein the first region of the hybridization probe comprises a moiety which inhibits the binding of the intact hybridization probe to the capture probe via steric hindrance.
wherein the capture probe more readily binds to the probe fragment than to the intact hybridization probe and wherein the hybridization probe is substantially single stranded at the Tₘ of the probe fragment/capture probe complex.

According to this disclosure, a method for detecting a target nucleic in a sample is provided which comprises:
melting the sample by heating the sample to a first temperature, wherein the sample comprises:
   a primer which hybridizes to at least a portion of the target nucleic acid;
   a hybridization probe comprising first and second regions, wherein the first region hybridizes to at least a portion of the target nucleic acid and the second region does not hybridize to the target nucleic acid and wherein the second region comprises a detectable label; and
   a polymerase and an enzyme comprising nuclease activity wherein the polymerase extends the hybridized primer in the direction of the hybridized probe and the nuclease activity of the enzyme cleaves the hybridized probe to thereby release a probe fragment comprising the second region of the probe and the detectable label; and
and wherein the first temperature is above the melting temperature (Tₘ) of the primer and double stranded nucleic acids present in the sample;
subsequently annealing the sample by reducing the temperature to a second temperature lower than the first temperature to allow the primer and the hybridization probe to each hybridize to a single stranded portion of the target nucleic acid in the sample; and
subsequently elongating the primer by allowing the polymerase to extend the primer hybridized to the target nucleic acid at a third temperature thereby releasing the probe fragment;
optionally repeating melting, annealing and elongating at least once;
contacting the sample with a surface of a solid support, wherein the surface of the solid support comprises one or more capture probes which hybridize to at least a portion of the second region of the probe fragment;
allowing the capture probe to hybridize to at least a portion of the probe fragments in the sample to form a probe fragment/capture probe complex at a fourth temperature lower than the second and third temperatures; and
detecting label on the surface of the solid support;
wherein the capture probe more readily binds to the probe fragment than to the intact hybridization probe and wherein the hybridization probe is substantially single stranded at the Tₘ of the probe fragment/capture probe complex.

According to another embodiment, a kit for detecting a target nucleic acid in a sample is provided which comprises:
(a) a hybridization probe comprising a first region which hybridizes to at least a portion of the target nucleic acid and a second region comprising a detectable label, wherein the second region does not hybridize to the target nucleic acid and wherein an enzyme comprising nuclease activity can cleave the hybridization probe when hybridized to the target nucleic acid to thereby produce a probe fragment comprising the second region and the detectable label;
(b) a solid support comprising one or more capture probes on a surface thereof, wherein the capture probe hybridizes to at least a portion of the second region of the probe fragment to form a probe fragment/capture probe complex, wherein the first region of the hybridization probe comprises a moiety which inhibits the binding of the intact hybridization probe to the capture probe via steric hindrance, and wherein the hybridization probe is substantially single stranded at the Tm of the probe fragment/capture probe complex;
(c) optionally, a primer which hybridizes to at least a portion of the target nucleic acid; and
(d) optionally, a polymerase which extends the hybridized primer in the direction of the hybridized probe and an enzyme comprising nuclease activity to thereby cleave the hybridized hybridization probe and release of the probe fragment comprising the second region of the probe and the detectable label.

### BRIEF DESCRIPTION OF THE DRAWINGS

*The skilled artisan will understand that the drawings, described below, are for illustration purposes only. The drawings are not intended to limit the scope of the present teachings in any way.*
FIG. 1 is a photograph of a multiplexed chip which can be used to detect nucleic acids in a sample.
FIG. 2 is a graph showing square wave voltammograms which illustrate the discrimination between positive samples and no template controls (NTC's) for Capture Probe 1, which has 25 bases between the hybridization region and the electrode surface, and Capture Probe 2, which has only 6 bases between the hybridization region and the electrode surface.
FIG. 3 is a bar graph of the electrochemical signal for the multiplexed chip modified with either Capture Probe 2 (3 runs) or Capture Probe 1 (2 runs) showing the improved discriminating capabilities of Capture Probe 2.
FIGS. 4A and 4B are schematic depictions illustrating the hybridization of cleaved and uncleaved probes to Capture Probe 1 (FIG. 4A) and Capture Probe 2 (FIG. 4B) illustrating the enhanced discrimination effect of Capture Probe 2.
FIGS. 5A-5C are bar graphs showing the results for hybridization of 19 mer, 15 mer and 13 mer hybridization probe fragments, respectively, to a 20 mer capture probe.
FIG. 6 is a schematic depiction of a electrochemical cell having a gold working electrode (WE) and a platinum counter electrode (CE).
FIG. 7 is a bar graph showing the signal generated for hybridization of three different hybridization probe/probe fragment combinations.
FIGS. 8A-8C are schematic depictions showing binding of the hybridization probe to the capture probe for the combinations used in FIG. 7.
FIG. 9 is a depiction of a scheme for the synthesis of an osmium complexing agent that can be coupled to the 5' amino group of a probe to form an Os-labeled probe.

### DETAILED DESCRIPTION

*For the purposes of interpreting of this specification, the following definitions will apply and whenever appropriate, terms used in the singular will also include the plural and vice versa. In the event that any definition set forth below conflicts with the usage of that word in any other document, the definition set forth below shall always control for purposes of interpreting this specification and its associated claims unless a contrary meaning is clearly intended (for example in the document where the term is originally used). The use of "or" herein means "and*/*or" unless stated otherwise or where the use of "and*/*or" is clearly inappropriate. The use of "a " herein means "one or more" unless stated otherwise or where the use of "one or more" is clearly inappropriate. The use of "comprise,*" *"comprises," "comprising" "include,"includes," and "including" are interchangeable and not intended to be limiting. Furthermore, where the description of one or more embodiments uses the term "comprising," those skilled in the art would understand that in some specific instances, the embodiment or embodiments can be alternatively described using language "consisting essentially of" and*/*or "consisting of.*

As used herein, "capture probe" refers to a nucleobase polymer that is surface bound. The capture probe can be a nucleic acid (e.g. DNA or RNA), a nucleic acid analog (e.g. locked nucleic acid (LNA)), a nucleic acid mimic (e.g. peptide nucleic acid (PNA)) or a chimera.

As used herein, "chimera" refers to a nucleobase polymer comprising two or more linked subunits that are selected from different classes of subunits. For example, a PNA/DNA chimera would comprise at least one PNA subunit linked to at least one 2'-deoxyribonucleic acid subunit (For exemplary methods and compositions related to PNA/DNA chimera preparation See: WO96/40709). Exemplary component subunits of a chimera are selected from the group consisting of PNA subunits, naturally occurring amino acid subunits, DNA subunits, RNA subunits, LNA subunits and subunits of other analogues or mimics of nucleic acids.

As used herein, "flap" refers to a portion of a hybridization probe that is non-complementary to the target nucleic acid the probe is designed to determine.

As used herein, "hybridization probe" is a nucleobase polymer that can be cleaved by nuclease activity of an enzyme at a site where the probe is hybridized to a complementary strand, said hybridization probe comprising a nucleobase sequence that is complementary to at least a portion of a target nucleic acid of interest in a sample. The hybridization probe can be a oligonucleotide, oligonucleotide analog or chimera so long as it is cleavable by nuclease activity. In some embodiments, the nucleobase polymer can be a chimera that comprises all DNA subunits except for one LNA subunit. In some embodiments, the nucleobase polymer comprises a single LNA subunit that is situated one subunit removed (toward the 3' end) from the 5' end of that portion of the hybridization probe that is designed to hybridize to the target nucleic acid.

As used herein, "nuclease activity" refers to the ability of an enzyme to cleave the backbone of a nucleobase polymer (e.g., a nucleic acid). Non-limiting examples of nuclease activity include exonuclease activity (i.e., the ability of an enzyme to cleave nucleotide sequences sequentially from the free end of a nucleobase polymer substrate) and endonuclease activity (i.e., the ability of a protein to recognize specific, short sequences of a nucleobase polymer and to cleave the nucleobase polymer at those sites).

As used herein, "nucleobase polymer" refers to a polymer comprising a series of linked nucleobase containing subunits. Non-limiting examples of suitable polymers include oligodeoxynucleotides, oligoribonucleotides, peptide nucleic acids, nucleic acid analogs, nucleic acid mimics and chimeras.

As used herein, "peptide nucleic acid" or "PNA" refers to any polynucleobase strand or segment of a polynucleobase strand comprising two or more PNA subunits, including, but not limited to, any polynucleobase strand or segment of a polynucleobase strand referred to or claimed as a peptide nucleic acid in United States Patent Nos. 5,539,082, 5,527,675, 5,623,049, 5,714,331, 5,718,262, 5,736,336, 5,773,571, 5,766,855, 5,786,461, 5,837,459, 5,891,625, 5,972,610, 5,986,053, 6,107,470 and 6,357,163. For the avoidance of any doubt, PNA is a nucleic acid mimic and not a nucleic acid or nucleic acid analog. PNA is not a nucleic acid since it is not formed from nucleotides. For the avoidance of doubt, PNA oligomers may include polymers that comprise one or more amino acid side chains linked to the backbone.

As used herein, "support", "solid support" or "solid carrier" refers to any solid phase material. Solid support encompasses terms such as "resin", "synthesis support", "solid phase", "surface" "membrane" and/or "support". A solid support can be composed of organic polymers such as polystyrene, polyethylene, polypropylene, polyfluoroethylene, polyethyleneoxy, and polyacrylamide, as well as co-polymers and grafts thereof. A solid support can also be inorganic, such as glass, silica, controlled-pore-glass (CPG), or reverse-phase silica. The configuration of a solid support can be in the form of beads, spheres, particles, granules, a gel, a membrane or a surface. Surfaces can be planar, substantially planar, or non-planar. Solid supports can be porous or non-porous, and can have swelling or non-swelling characteristics. A solid support can be configured in the form of a well, depression, tube, channel, cylinder or other container, vessel, feature or location.

As used herein, "target nucleic acid" refers to a nucleic acid molecule of interest. A sample can comprise more than one target nucleic acid molecule.

Methods for electrochemically monitoring the outcome of a PCR reaction are disclosed in U.S. Patent Application No. 11/488,439, filed on July 17, 2006. This application describes assays which employ a hybridization probe which, upon cleavage, yields a cleavage product that is a single-stranded oligonucleotide that can hybridize at a modifed electrode surface for detection. This type of detection involves separation of the cleaved probe from the uncleaved probe. Separation, for example, can be accomplished by capture of a biotin labeled probe on a streptavidin matrix.

A United States patent application with publication number US 2005/0255512 discloses a method of detecting or measuring a target nucleic acid in a sample. The method includes forming a cleavage structure by incubating the sample with a probe. The probe has a binding moiety secondary and a structure that changes upon binding to the target nucleic acid. The method includes cleaving the cleavage structure with a nuclease to release a nucleic acid fragment to generate a signal and thus indicating the presence of the target nucleic acid. The method further includes detecting or measuring the amount of the fragment captured by binding of the binding moiety to a capture element on a solid support.

An international application with publication number WO 2001/06016 discloses a method for detecting a target nucleic acid sequence. The method includes hybridizing a primer to the target nucleic acid sequence to form a hybridization complex and contacting the complex with an enzyme to form a modified primer nucleic acid. The complex is disassociated and an assay complex is formed with at least one electron transfer moiety and the modified primer nucleic acid. The assay complex is covalently attached to an electrode and electron transfer between the electron transfer moiety and the electrode is then detected as an indication of the presence of the target nucleic acid sequence.

An international application with publication number WO 2005/010199 discloses a further method for detecting target nucleotide sequences. The method uses at least one invasive cleavage reaction to generate tagged molecules having identifier tags corresponding to target nucleotide sequences. The hybridization of any tagged molecule with a complementary detection probe on a universal detector then indicates the presence of the corresponding target in the sample being assayed.

The present inventors have discovered that the ability to discriminate between cleaved and intact hybridization probes can also be achieved without separation by modifying the structure of the solid phase capture probe and/or the hybridization probe. In particular, it has been discovered that the structure of the solid phase capture probe and/or the hybridization probe has an affect on the ability of the capture probe to discriminate between the intact hybridization probe and the cleaved probe fragment. While not wishing to be bound by theory, it is believed that this phenomenon results from a steric hindrance effect that inhibits hybridization of the intact or uncleaved (i.e., the longer or more bulky) hybridization probe to the capture probe.

According to this disclosure, the hybridization probe is substantially single stranded at the Tₘ of the probe fragment/capture probe complex wherein "substantially single stranded" means that less than 5% of the hybridization probe is part of a double stranded complex (e.g., a folded structure).

### Electrode Surface Capture Probes

Two electrode capture probe sequences were investigated for their ability to discriminate between the cleaved hybridization probe fragment and the intact hybridization probe. The capture probe sequences employed in the following experiments differ by the distance of nineteen bases between the hybridization region, which is shown in boldface and underlined below, and the gold surface. The sequences of the two capture probes are shown below, with the portion of the sequence homologous to the second region of the probe fragment(s) produced by cleavage of the hybridization probe shown in bold and underlined.
Capture Probe 1: 
5' (DTPA)(DTPA)(DTPA) AAA AAA ACC CCA GCA ATT CAA GTG TGT TGA

GAG CTT TGA T 3'

(SEQ ID NO: 1)
Capture Probe 2:
   5' (DTPA)(DTPA)(DTPA) AAA AAA **TTG AGA GCT TTG AT**T CGT G 3' (SEQ ID NO: 2)

The oligonucleotides were modified on the 5' end with the dithiol phosphoramidite (DTPA) (Glen Research, Inc) for attachment to the gold electrodes. The capture probes were attached to the electrodes using the following procedure. First, the electrodes were cleaned by exposure to an UV Ozone Cleaner (Jelight Inc) for 20 min followed by an ethanol soak to reduce the oxide formed. Then, 40 µL of a 1 uM solution of the thiolated capture probe in 1M Phosphate buffer (pH 7) was deposited on the surface for 15 min in an electrode area defined by a silicone well (Molecular Probes, Inc). The electrodes were then rinsed in water and exposed to a 2 mM mercaptohexanol solution for 2 hrs. After exposure, the electrodes were rinsed in water and dried under argon.

### PCR hybridization Probe

The PCR hybridization probe was obtained from IDT Inc. with a 5' amine modification so that it could be coupled in-house to an electroactive Ferrocene (Fc) moiety. The sequence is as follows with the 5' flap indicated in bold and underlined:
5' Fc **-ATC AAA GCT CTC A**AC GCC TGC AAG TCC TAA GAC GCC A- biotin (SEQ ID NO: 3)
The biotin modification on the 3' end was not utilized in the following experiments.

### PCR Conditions for Listeria

PCR was performed in 1X buffer A from core PCR kit (Applied Biosystems Ca# N808-0228) supplemented with 6 mM MgCl₂. PCR primers and Ferrocene labeled hybridization probe were present at concentrations 200 nM and 400 nM, respectively. The 25 µL, reaction mix contained either 3000 or 0 copies of Listeria DNA for positive and negative (i.e., no template control) samples. Cycling parameters were as follows: 95° C for 10 min., then (95° C for 15 sec and 66° C for 30 sec) X 40 cycles.
Immediately after PCR, the 25 µL reaction mix was placed on the gold electrode and covered with a glass coverslip for I hr static hybridization at room temperature. Alternatively, the PCR mix was introduced into the multiplexed electrode chip for flow-through detection.

### Multiplexed Electrode Chip

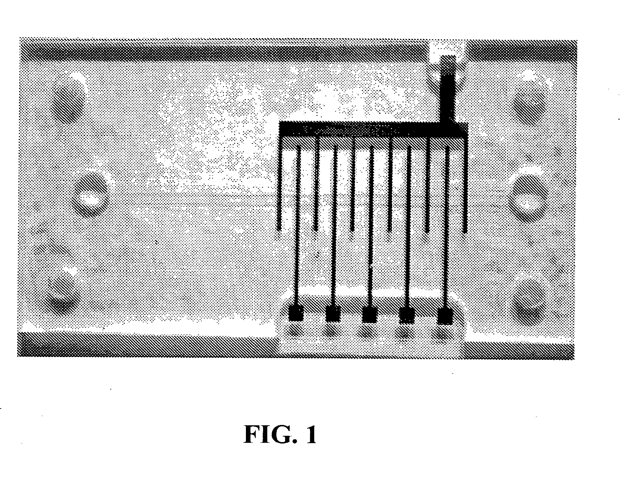

FIG. 1 is a photograph of a multiplexing chip which can be used for electrochemical measurements. As shown in FIG. 1, the chip includes 500 µm wide gold finger working electrodes inter-digitated with a pronged counter electrode crossing a flow channel of 350 µm width and 120 µm height. The gold electrodes are modified before assembly with the indicated capture probes and then the chip is assembled. Subsequently, 20 µL of the completed PCR solutions are flowed through the chip at a flow rate of 1 µL/ min to allow for hybridization.

All electrochemical measurements are performed as described previously.

### Results

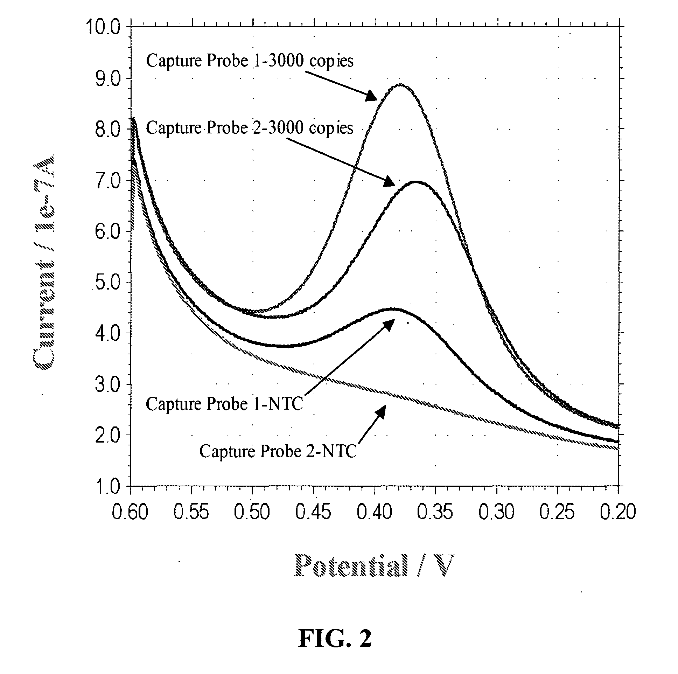

FIG. 2 shows the results for the planar gold electrode with a static, I hour hybridization. As can be seen from the data in FIG. 2, discrimination between the positive sample and the no template control (NTC) can be seen for both capture probes. However for Capture Probe 2, there is much lower signal from the NTC sample. This indicates that the intact hybridization probe does not hybridize well to that probe surface.

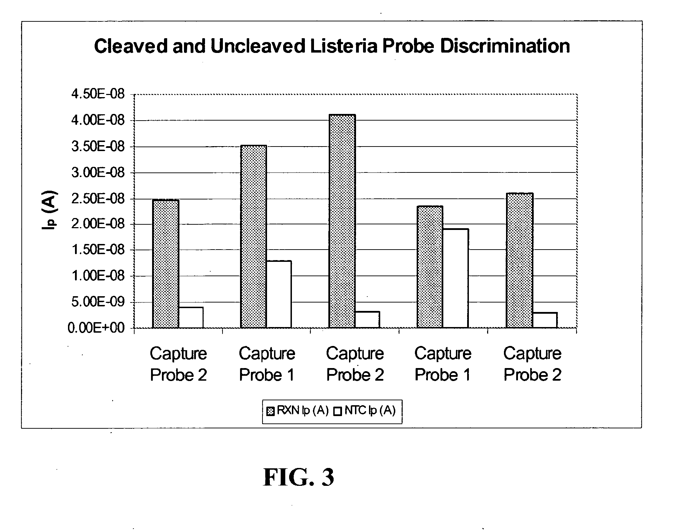

The results obtained using the multiplexed, flow through chip of FIG. 1 are set forth in FIG. 3. The data depicted in FIG. 3 is summarized in the table below.

| | RXN | | NTC | | Dis. Ratio |
|---|---|---|---|---|---|
| | Iₚ(A) | A (VA) | Iₚ(A) | A (VA) | Rxn/NTC |
| Capture Probe 2 | 2.48E-08 | 2.40E-09 | 3.90E-09 | 4.02E-10 | 6.4 |
| Capture Probe 1 | 3.51E-08 | 3.57E-09 | 1.30E-08 | 1.14E-09 | 2.7 |
| Capture Probe 2 | 4.11 E-08 | 4.20E-09 | 3.08E-09 | 3.40E-10 | 13.3 |
| Capture Probe 1 | 2.34E-08 | 2.25E-09 | 1.90E-08 | 1.76E-09 | 1.2 |
| Capture Probe 2 | 2.60E-08 | 2.71E-09 | 2.94E-09 | 2.90E-10 | 8.8 |

wherein "Dis. Ratio" represents the discrimination ratio of the capture probes.

For purposes of the present application, the "discrimination ratio" of the capture probe is the ratio obtained by dividing the intensity of the signal generated from the positive sample by the intensity of the signal generated by the no template control (NTC) under the conditions and using the protocol set forth above.

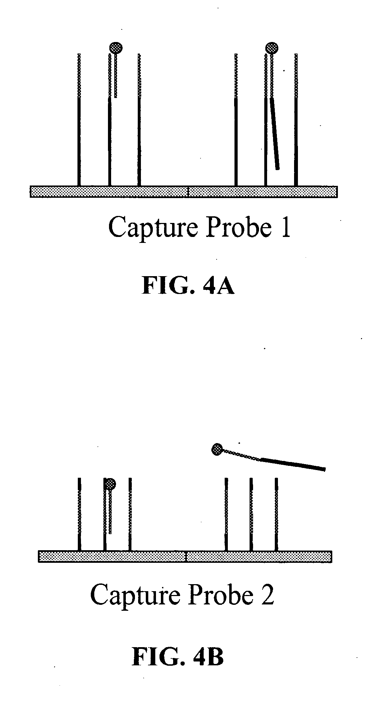

While not wishing to be bound by any theory, it is believed that the shorter capture probe which has a hybridization region closer to the surface of the electrode prevents efficient hybridization of the full length, intact hybridization probe to the capture probe. This steric hindrance effect is illustrated in FIGS. 4A and 4B. In particular, FIG. 4A illustrates the hybridization of cleaved and intact hybridization probes to the longer Capture Probe 1 and FIG. 4B illustrates the hybridization of cleaved and intact hybridization probes to the shorter Capture Probe 2.

The ability of the capture probe to discriminate between the probe fragment and the intact hybridization probe can also be enhanced by variations in said hybridization probe. In particular, the present inventors have discovered that the length of the 5' flap of the hybridization probe (which 5' flap is part of the probe fragment after cleavage of the hybridization probe by the nuclease activity of the enzyme) also influences the ability of the capture probe to discriminate between cleaved and intact hybridization probe.

To demonstrate this phenomenon, a bird flu assay was conducted using the following hybridization probes:
19-mer 5' flap
   **GTTACTTCGTTCGATTGT**C^{▼}TGGACTTATAATGCTGAACTTCTGGT (SEQ ID NO: 4)
15-mer 5' flap
   **CTTCGTTCGATTGT**C^{▼}TGGACTTATAATGCTGAACTTCTGGT (SEQ ID NO: 5)
13-mer 5' flap
   **TCGTTCGATTGT**C^{▼}TGGACTTATAATGCTGAACTTCTGGT (SEQ ID NO: 6)
The nucleobases illustrated above in bold in these sequences represent the 5' flap and the ^{▼} symbol represents the site where cleavage by the exonucleoase activity is expected to be predominant. In these hybridization probes, the underlined C nucleobase that is adjacent to the illustrated cleavage site is an LNA subunit. All other subunits of these hybridization probes are DNA.

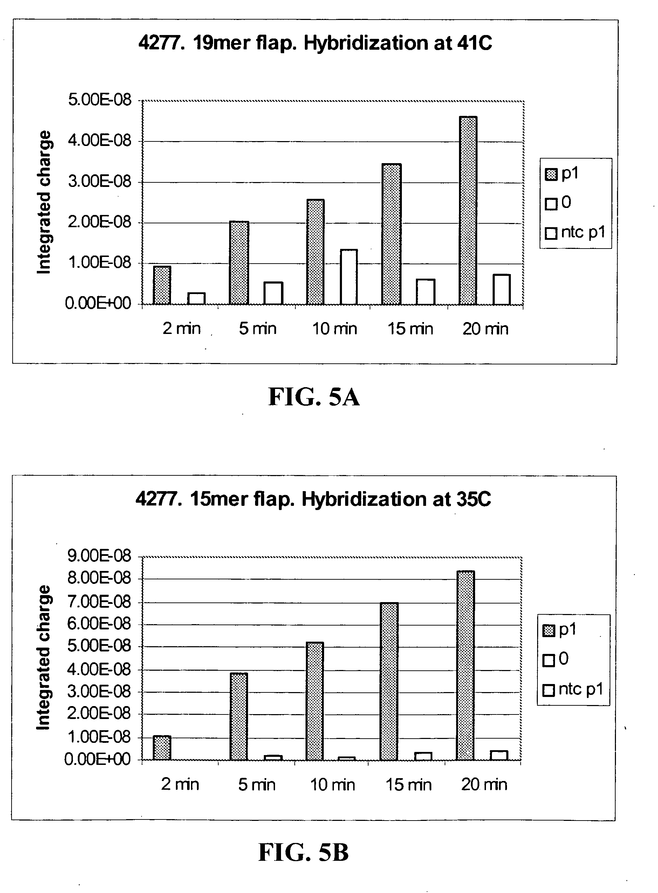

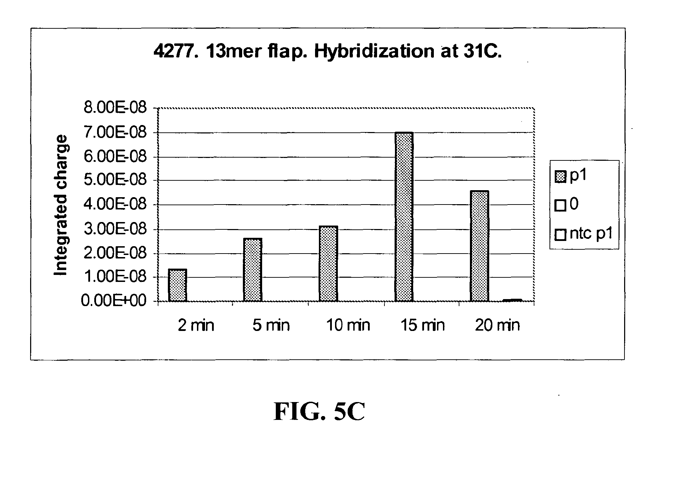

When cleaved by the nuclease activity of the enzyme, the probe of SEQ ID NO: 4 produces, when cleaved, a probe fragment comprising the 19 mer 5' flap whereas the probe of SEQ ID NO: 5 produces a probe fragment comprising the 15 mer 5' flap and the probe of SEQ ID NO: 6 produces a probe fragment comprising the 13 mer 5' flap. Bar graphs showing the results for hybridization to a 20 mer capture probe are provided in FIGS. 5A-5C for the each of the probe fragments comprising the 19 mer, 15 mer and 13 mer 5' flaps, respectively. As can be seen from these charts, the probe fragment comprising the 13 mer flap produces much higher discrimination between the cleaved and intact hybridization probe than do the probe fragments comprising longer flaps. Thus, it seems that shortening of the 5' flap decreases the binding of the intact hybridization probe to the surface bound capture probe. For the data in FIGS. 5A-5C, hybridizations were carried out at temperatures 10° C below the predicted Tₘ of the duplexes formed by the second region (i.e. the nucleobase sequence of the 5' flap) of the probe fragments and the capture probe.

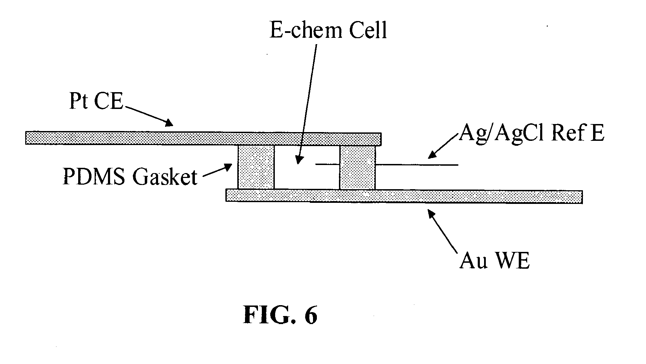

FIG. 6 is a schematic depiction of an electrochemical cell having a gold working electrode (WE) and a platinum counter electrode (CE) that can be used in the above described assays. As shown in FIG. 6, the electrochemical cell is formed by sandwiching a PDMS gasket between the counter and working electrodes. The working electrode (WE) and the counter electrode (CE) can have diameters of 2 mm. The platimun counter-electrode (CE) can be made by sputter coating a 2000 Angstrom thick platinum layer on a silicon wafer having a Cr adhesion layer. The gold counter-electrode (CE) can be made by sputter coating a 2000 Angstrom thick gold layer on a silicon wafer having a Cr adhesion layer. The reference electrode can be a 0.5 mm diameter Ag/AgCl wire.

### Detection of Cleaved Tag in the Presence of Uncleaved Probe

This example illustrates embodiments in which a tag complement is immobilized on an electrode by thiol moieties (here provided by DTPA moieties) that exhibit specificity for binding to gold surfaces, such as a gold electrode, and a cleavable probe that contains (i) a polynucleotide sequence attached to the 5' end of a target complementary segment and (ii) a detectable tag comprising an osmium-containing complex for electrochemical detection after capture of the cleaved tag by the immobilized tag complement.

The cleaved probe can be detected and/or measured in the presence of uncleaved probe by selection of an appropriate capture probe (a tag complement) such that the capture probe destabilizes capture of uncleaved (intact) probe by selectively binding the tag of the uncleaved probe close to the electrode surface. As a result, the capture probe hybridizes to the cleaved tag more stably than the uncleaved tag moiety bound to the probe.

A 50 µl reaction mix is prepared that contains 1X PCR buffer A (Applied Biosystems, P/N N808-0228), 6 mM MgCl₂, 200 µM of each dNTP, 200 nM of forward and reverse primers, 400 nM 5'-Os-labeled probe, 0.05 units of Gold AmpliTaq™ polymerase and 3,000 copies of *Listeria monocytogenesis* DNA.

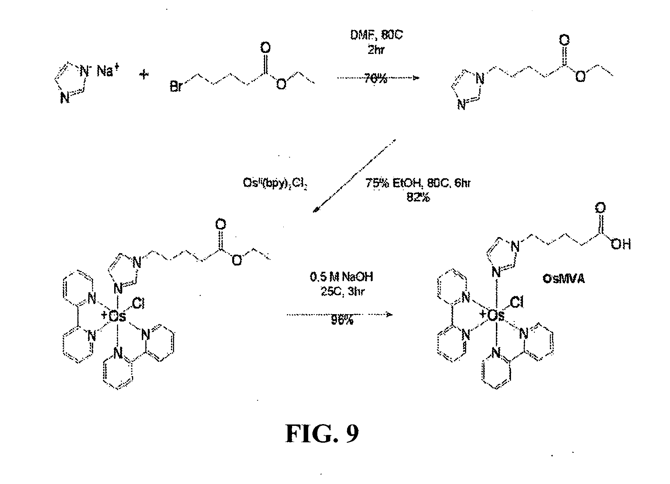

The osmium complex labeling agent that was coupled to the 5' amino group of each probe to form the Os-labeled probe is shown in FIG. 9 along with a scheme for the synthesis of the osmium complexing agent. The forward and reverse primers used during the PCR were as follows:
5'-CATGGCACCACCAGCATCT SEQ ID NO: 7
   and
5'-ATCCGCGTGTTTCTTTTCGA SEQ ID NO: 8

Three different combinations of cleavable probes and immobilized tag complements were tested, as shown in the following combinations in which the upper sequence (underlined) represents the Os-labeled cleaved tag to be detected, and the lower sequence represents a capture probe that was attached to the electrode by 3 DTPA moieties at its 5' end, and contained a tag complement for binding to the tag sequence:

| | | |
|---|---|---|
| Combination #1 | | |
| Tag 1: | 5'-CACGAATCAAAGCTCTCAAX-3' | SEQ ID NO: 9 |
| Cap1 : | 3'-GTGCTTAGTTTCGAGAGTTGTGTGAACTTAACGACCCCAAAAAAA5' | SEQ ID NO: 10 |
| Combination #2 | | |
| Tag 1: | 5'-CACGAATCAAAGCTCTCAAX-3' | SEQ ID NO: 11 |
| Cap2: | 3'-AAAAAAGTGCTTAGTTTCGAGAGTT(C18)5' | SEQ ID NO: 12 |

| Combination #3 | | |
|---|---|---|
| Tag 2: | 5'-ATCAAAGCTCTCAAX-3' | SEQ ID NO: 13 |
| Cap2: | 3' AAAAAAGTGCTTAGTTTCGAGAGTT(C18)5' | SEQ ID NO: 14 |

wherein X is:
CGCCTGCAAGTCCTAAGACGCCA-3' (target-specific segment) SEQ ID NO: 15 and C18 is (OCH₂CH₂)₆(DTPA)₃

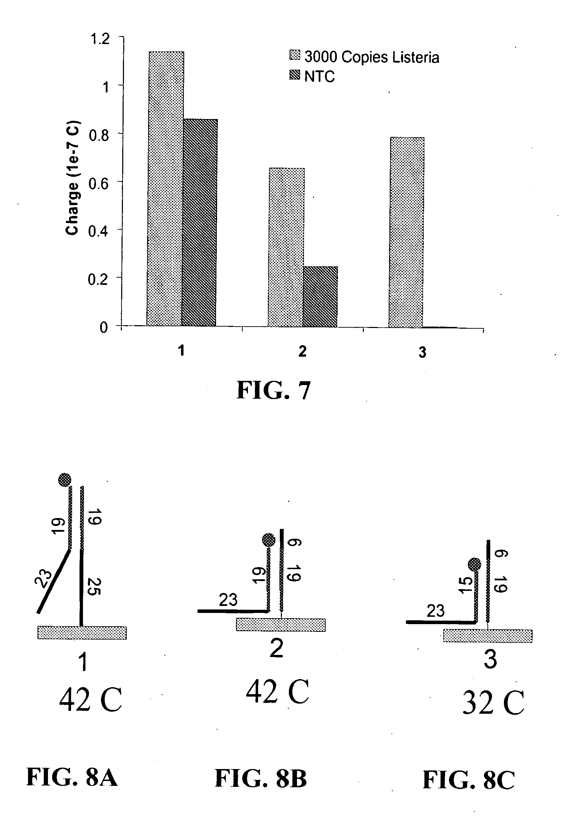

Thermocycling was performed at 95°C for 10 min., then (92° C for 15 sec, 66° C for 30 sec.) X 40 cycles. Then, the PCR mix was loaded into an electrochemical cell of the type depicted in FIG. 6 for electrochemical measurements. The measurements were performed using a 1 M NaCl hybridization buffer at 31°C (which is approximately 10 degrees below the melt temperature (Tₘ) of the 15-mer cleaved tag sequence in Combination #3 above as calculated using the Tₘ calculator program on IDT web site: www.idt.com). Results are shown in FIG. 7.

FIG. 7 is a bar graph showing the results for hybridization of the three different hybridization probe/probe fragment combinations set forth above. A schematic depiction of the binding of the intact hybridization probes to the capture probe for each of the combinations is shown in FIGS 8A, 8B and 8C. As can be seen from FIGS. 8A-8C, each hybridization probe had a 23 mer region which did not hybridize to the capture probe. The hybridization probes used in Combinations 1 and 2 also had a 19 mer region (i.e. a 5 'flap) that hybridized to the capture probe whereas the hybridization probe used in Combination 3 had a shorter 15 mer region (i.e. 5' flap) that hybridized to the capture probe. The capture probe used in Combination 1 had a 25 mer spacer region between the support surface and the region that hybridizes to the hybridization probe. In contrast, the capture probes used in Combinations 2 and 3 did not have the 25 mer spacer region between the support surface and the region that hybridizes to the 5' flap of the hybridization probe. As can be seen from FIG. 7, Combination 3 provided by far the highest level of discrimination between the cleaved hybridization probe fragment and the intact hybridization probe. The hybridization probe used in Combination 3 had the shortest region which hybridized to the capture probe (15 mers). For Combinations 1 and 2, hybridization to the capture probe was conducted at 42° C whereas for Combination 3, hybridization was conducted to 32° C.

As set forth above, the ability of the capture probe to discriminate between the probe fragment and the intact hybridization probe can be enhanced by variations in both the sequence and length of the capture probe as well as by modifications of the hybridization probe. The hybridization probe can also be modified by extending the 3' end of the hybridization probe or by adding a bulky modification on the 3' end of the hybridization probe that would further block access to the capture probe sequence on the solid support surface.

Any known electrochemical moiety can be used as a label on the cleaved portion of the hybridization probe. Exemplary electrochemical labels which may be used include bis(2,2'-bipyridyl)imidizolylchloroosmium(II) [salt]. This label gives a good Eₒ of 0.165 vs Ag/AgCl and has good solubility properties for synthesis and purification. Other exemplary labels include ferrocene as well as the labels disclosed in U.S. Patent Application No. 11/488,439 filed on July 17, 2006. Moreover, the electrochemical label can be any moiety that can transfer electrons to or from an electrode. Exemplary electrochemical labels include transition metal complexes. Suitable transition metal complexes include, for example, ruthenium²⁺ (2,2'-bipyridine)₃ (Ru(bpy)₃²⁺), ruthenium²⁺(4,4'-dimethyl-2,2'-bipyridine)₃ (Ru(Me²-bpy)₃²⁺), ruthenium²⁺(5,6-dimethyl-1,10-phenanthroline)₃ (Ru(Me₂-phen)₃²⁺), iron²⁺(2,2'-bipyridine)₃ (Fe(bpy)₃²⁺), iron²⁺(5-chlorophenanthroline)₃ (Fe(5-Cl-phen)₃²⁺), osmium²⁺(5-chlorophenanthroline)₃ (Os(5-Cl-phen)₃²⁺), osmium²⁺(2,2'-bipyridine)₂ (imidazolyl), dioxorhenium¹⁺ phosphine, and dioxorhenium¹⁺ pyridine (ReO₂ (py)₄¹⁺). Some anionic complexes useful as mediators are: Ru(bpy)((SO₃)₂-bpy)₂²⁻ and Ru(bpy)((CO₂)₂-bpy)₂²⁻ and some zwitterionic complexes useful as mediators are Ru(bpy)₂ ((SO₃)₂-bpy) and Ru(bpy)₂((CO₂)₂-bpy) where (SO₃)₂-bpy₂- is 4,4'-disulfonato-2,2'-bipyridine and (CO₂)₂-bpy₂- is 4,4'-dicarboxy-2,2'-bipyridine. Suitable substituted derivatives of the pyridine, bypyridine and phenanthroline groups may also be employed in complexes with any of the foregoing metals. Suitable substituted derivatives include but are not limited to 4-aminopyridine, 4-dimethylpyridine, 4-acetylpyridine, 4-nitropyridine, 4,4'-diamino-2,2'-bipyridine, 5,5'-diamino-2,2'-bipyridine, 6,6'-diamino-2,2'-bipyridine, 4,4'-diethylenediamine-2,2'-bipyridine, 5,5'-diethylenediamine-2,2'-bipyridine, 6,6'-diethylenediamine-2,2'-bipyridine, 4,4'-dihydroxyl-2,2'-bipyridine, 5,5'-dihydroxyl-2,2'-bipyridine, 6,6'-dihydroxyl-2,2'-bipyridine, 4,4', 4"-triamino-2,2',2"-terpyridine, 4,4',4"-triethylenediamine-2,2',2"-terpyridine, 4,4',4"-trihydroxy-2,2',2'-terpyridine, 4,4',4"-trinitro-2,2',2"-terpyridine, 4,4',4"-triphenyl-2,2',2"-terpyridine, 4,7-diamino-1,10-phenanthroline, 3,8-diamino-1,10-phenanthroline, 4,7-diethylenediamine-1,10-phenanthroline, 3,8-diethylenediamine-1,10-phen anthroline, 4,7-dihydroxyl-1,10-phenanthroline, 3,8-dihydroxyl-1,10-phenanthroline, 4,7-dinitro- 1,10-phenanthroline, 3,8-dinitro-1,10-phenanthroline, 4,7-diphenyl-1,10-phenanthroline, 3,8-diphenyl- 1,10-phenanthroline, 4,7-disperamine-1,10-phenanthroline, 3,8-disperamine-1,10-phenanthroline, dipyrido[3,2-a:2',2'-c]phenazine, and 6,6'-dichloro-2,2'-bipyridine, among others.

In addition, although electrochemical detection is exemplified above, the disclosed methods are also applicable to the detection of nucleic acids by other detection techniques, such as fluorescence detection. Moreover, the detectable label on the hybridization probe can be any moiety which is capable of being detected and/or quantitated. Exemplary labels include electrochemical, luminescent (e.g., fluorescent, luminescent, or chemiluminescent) and colorimetric labels.

The primers and probes used herein may have any of a variety of lengths and configurations. For example, the primers may be from 18 to about 30 subunits in length or from 20 to 25 subunits in length. Longer or shorter length primers can also be used. The length of the region of the hybridization probe which binds to the target nucleic acid can be from 8 to 30 subunits whereas the length of the region of the hybridization probe which does not bind to the target can have a length of 2 to 40 subunits or from 8 to 30 subunits. Hybridization probes having longer or shorter regions than those exemplified above can also be used.

The primers (e.g., PCR primers) may be designed to bind to and produce an amplified product of any desired length, usually at least 30 or at least 50 nucleotides in length and up to 200, 300, 500, 1000, or more nucleotides in length. The probes and primers may be provided at any suitable concentrations. For example, forward and reverse primers for PCR may be provided at concentrations typically less than or equal to 500 nM, such as from 20 nM to 500 nm, or 50 to 500 nM, or from 100 to 500 nM, or from 50 to 200 nM. Probes are typically provided at concentrations of less than or equal to 1000 nM, such as from 20 nM to 500 nm, or 50 to 500 nM, or from 100 to 500 nM, or from 50 to 200 nM. Exemplary conditions for concentrations of NTPs, enzyme, primers and probes can also be found in U.S. Patent No. 5,538,848 or can be achieved using commercially available reaction components (e.g., as can be obtained from Applied Biosystems, Foster City, CA).

A plurality of complementary capture probes, each having a characteristic sequence, may also be used. For example, an array of capture oligonucleotides that hybridize to different hybridization probe fragments may be used to localize and capture individual tag sequences in a plurality of discrete detection zones.

The methods described herein can be used to detect target nucleic acid in real time. For example, the solid support can be in contact with the solution in which nucleic acid amplification is occurring and the process monitored during PCR (i.e. real-time detection). Alternatively, detection of probe fragments can also be conducted after the amplification process is complete (i.e., end-point detection). In some embodiments, the PCR assay can be monitored during PCR (real-time) and after the process is completed (i.e. end-point). PCR assays can be performed using traditional PCR formats as well as Fast PCR formats, asymmetric PCR formats and asynchronous PCR formats.

## Claims

1. A method of detecting a target nucleic acid in a sample, the method comprising:
(a) incubating the sample with:
(1) a primer which hybridizes to at least a portion of the target nucleic acid;
(2) a hybridization probe comprising first and second regions, wherein the first region hybridizes to at least a portion of the target nucleic acid and the second region does not hybridize to the target nucleic acid, the second region comprising a detectable label; and
(3) a polymerase and an enzyme comprising an nuclease activity, wherein the polymerase extends the hybridized primer in the direction of the hybridized probe and the nuclease activity of the enzyme cleaves the hybridized probe to thereby release a probe fragment comprising the second region and the detectable label;
(b) allowing the primer and the hybridization probe to hybridize to target nucleic acid in the sample;
(c) allowing the polymerase to extend the hybridized primer;
(d) allowing the nuclease activity of the enzyme to cleave the hybridized hybridization probe to thereby release the probe fragment;
(e) contacting the sample with a surface of a solid support, wherein the surface of the solid support comprises one or more capture probes each of which hybridizes to at least a portion of the second region of the probe fragment;
(f) allowing the capture probes to hybridize to probe fragment in the sample to form a probe fragment/capture probe complex; and
(g) detecting the label on the surface of the solid support,
wherein the hybridization probe is substantially single stranded at the Tm of the probe fragment/capture probe complex, and wherein the first region of the hybridization probe comprises a moiety which inhibits the binding of the intact hybridization probe to the capture probe via steric hindrance.

2. The method of claim, 1, wherein the second region of the probe fragment binds to the capture probe such that the portion of the second region adjacent the first region in the intact hybridization probe is oriented toward the solid support surface, wherein, optionally, a proximal region of the capture probe adjacent to the solid support surface does not hybridize to the probe fragment and a distal region of the capture probe away from the solid support surface hybridizes to the second region of the probe fragment.

3. The method of Claim 2, wherein the proximal region of the capture probe is shorter than the first region of the hybridization probe.

4. The method of Claim 1, wherein the capture probe and the hybridization probe each comprise polynucleotides, wherein the polynucleotides optionally comprise deoxyribonucleotides.

5. The method of Claim 4, wherein the proximal region of the capture probe has fewer nucleotides than the first region of the intact hybridization probe.

6. The method of Claim 1, wherein the hybridization probe further comprises a third region adjacent the first region and opposite the second region, wherein the third region does not hybridize to the target nucleic acid.

7. The method of Claim 1, comprising:
(a) melting the sample by heating the sample to a first temperature wherein the first temperature is above the melting temperature (Tm) of the primer and double stranded nucleic acids are present in the sample that comprise the target nucleic acid;
(b) subsequently annealing the sample by reducing the temperature to a second temperature lower than the first temperature to allow the primer and the hybridization probe to each hybridize to a single stranded portion of the target nucleic acid in the sample; and
(c) subsequently elongating the primer by allowing the polymerase to extend the primer hybridized to the target nucleic acid at a third temperature thereby releasing the probe fragment;
(d) optionally repeating melting, annealing and elongating at least once;
(e) contacting the sample with a surface of a solid support, wherein the surface of the solid support comprises one or more capture probes which hybridize to at least a portion of the second region of the probe fragment;
(f) allowing the capture probes to hybridize to at least a portion of the probe fragment in the sample to form a probe fragment/capture probe complex at a fourth temperature lower than the second and third temperatures; and
(g) detecting label on the surface of the solid support.

8. The method of any preceding claim, wherein the capture probe has a discrimination ratio of 3 or greater.

9. The method of Claim 7, wherein the second temperature and the third temperature are the same.

10. The method of any preceding claim, wherein the polymerase and the enzyme comprising the nuclease activity are the same molecule, wherein the polymerase is optionally a thermostable enzyme.

11. The method of any preceding claim, wherein the surface of the solid support comprises an electrode and wherein the detectable label is a moiety that can transfer electrons to or from the electrode, wherein the detectable label is optionally a Ferrocene moiety, and/ or wherein the surface of the solid support optionally comprises gold.

12. The method of any preceding claim, wherein the solid support comprises a plurality of interdigitated plates forming a flow channel, wherein at least some of the surfaces of the plates comprise capture probes, and wherein contacting the sample with a surface of a solid support comprises flowing the sample through the flow channel, wherein the surfaces of the plates optionally comprise electrodes, and/ or wherein the surfaces of alternating plates optionally comprise capture probes.

13. The method of Claim 7, wherein melting, annealing and elongating are performed multiple times in a series of cycles, wherein detecting label on the surface of the solid support optionally occurs after the last melting, annealing and elongating cycle.

14. The method of Claim 7, wherein the sample is in contact with the surface of the solid support during melting, annealing and elongating, and wherein detecting occurs multiple times during the method and/or after the last melting, annealing and elongation cycle.

15. A kit for detecting a target nucleic acid in a sample comprising:
(a) a hybridization probe comprising a first region which hybridizes to at least a portion of the target nucleic acid and a second region comprising a detectable label, wherein the second region does not hybridize to the target nucleic acid and wherein an enzyme comprising nuclease activity can cleave the hybridization probe when hybridized to the target nucleic acid to thereby produce a probe fragment comprising the second region and the detectable label;
(b) a solid support comprising one or more capture probes on a surface thereof, wherein the capture probe hybridizes to at least a portion of the second region of the probe fragment to form a probe fragment/capture probe complex, wherein the first region of the hybridization probe comprises a moiety which inhibits the binding of the intact hybridization probe to the capture probe via steric hindrance, and wherein the hybridization probe is substantially single stranded at the Tm of the probe fragment/capture probe complex;
(c) optionally, a primer which hybridizes to at least a portion of the target nucleic acid; and
(d) optionally, a polymerase which extends the hybridized primer in the direction of the hybridized probe and an enzyme comprising nuclease activity to thereby cleave the hybridized hybridization probe and release of the probe fragment comprising the second region of the probe and the detectable label.

16. The kit of Claim 15, wherein the capture probe has a discrimination ratio of 3 orgreater.

17. The kit of Claim 15, wherein the surface of the solid support comprises an electrode and wherein the detectable label is a moiety that can transfer electrons to or from the electrode, preferably wherein the detectable label is optionally an electroactive Ferrocene moiety, and! or wherein the surface of the solid support optionally comprises gold.

18. The kit of claim 15, wherein the polymerase and the enzyme comprising nuclease activity are the same molecule.

19. The method of any of Claims 1-14, or the kit according to any of Claims 15-18, wherein the nuclease activity is exonuclease activity.

20. The method of claim 8, wherein the capture probe has a discrimination ratio of 5 or greater.

21. The method of claim 10, wherein the polymerase is Taq polymerase.

22. The kit of claim 16, wherein the capture probe has a discrimination ratio of 5 or greater.

## Patentansprüche

1. Verfahren zum Nachweisen einer Zielnukleinsäure in einer Probe, wobei das Verfahren umfasst:
(a) Inkubieren der Probe mit:
(1) einem Primer, der mit zumindest einem Abschnitt der Zielnukleinsäure hybridisiert;
(2) eine Hybridisierungssonde, die erste und zweite Regionen umfasst, wobei die erste Region mit zumindest einem Abschnitt der Zielnukleinsäure hybridisiert, und wobei die zweite Region nicht mit der Zielnukleinsäure hybridisiert, wobei die zweite Region eine nachweisbare Markierung umfasst; und
(3) eine Polymerase und ein Enzym mit Nukleaseaktivität, wobei die Polymerase den hybridisierten Primer in Richtung der hybridisierten Sonde erweitert und die Nukleaseaktivität des Enzyms die hybridisierte Sonde spaltet, um ein Sondenfragment freizusetzen, das die zweite Region und die nachweisbare Markierung umfasst;
(b) Ermöglichen, dass der Primer und die Hybridisierungssonde mit der Zielnukleinsäure in der Probe hybridisieren;
(c) Ermöglichen, dass die Polymerase den hybridisierten Primer erweitert;
(d) Ermöglichen, dass die Nukleaseaktivität des Enzyms die hybridisierte Hybridisierungssonde spaltet, um das Sondenfragment freizusetzen;
(e) Inkontaktbringen der Probe mit einer Oberfläche eines Feststoffträgers, wobei die Oberfläche des Feststoffträgers eine oder mehrere Fängersonden umfasst, wobei jede davon mit zumindest einem Abschnitt der zweiten Region des Sondenfragments hybridisiert;
(f) Ermöglichen, dass die Fängersonden mit dem Sondenfragment in der Probe hybridisieren, um einen Sondenfragment/Fängersonde-Komplex zu bilden; und
(g) Nachweisen der Markierung auf der Oberfläche des Feststoffträgers,
wobei die Hybridisierungssonde bei der Tm des Sondenfragment/Fängersonde-Komplexes im Wesentlichen einzelsträngig ist, und wobei die erste Region der Hybridisierungssonde eine Einheit umfasst, die die Bindung der intakten Hybridisierungssonde an die Fängersonde durch sterische Hinderung hemmt.

2. Verfahren nach Anspruch 1, wobei die zweite Region des Sondenfragments an die Fängersonde bindet, so dass der Abschnitt der zweiten Region benachbart der ersten Region in der intakten Hybridisierungssonde hin zur Oberfläche des Feststoffträgers ausgerichtet ist, wobei optional eine proximale Region der Fängersonde benachbart der Oberfläche des Feststoffträgers nicht mit dem Sondenfragment hybridisiert und eine distale Region der Fängersonde weg von der Oberfläche des Feststoffträgers mit der zweiten Region des Sondenfragments hybridisiert.

3. Verfahren nach Anspruch 2, wobei die proximale Region der Fängersonde kürzer als die erste Region der Hybridisierungssonde ist.

4. Verfahren nach Anspruch 1, wobei die Fängersonde und die Hybridisierungssonde jeweils Polynukleotide umfassen, wobei die Polynukleotide optional Desoxyribonukleotide umfassen.

5. Verfahren nach Anspruch 4, wobei die proximale Region der Fängersonde weniger Nukleotide als die erste Region der intakten Hybridisierungssonde aufweist.

6. Verfahren nach Anspruch 1, wobei die Hybridisierungssonde ferner eine dritte Region benachbart der ersten Region und gegenüber der zweiten Region umfasst, wobei die dritte Region nicht mit der Zielnukleinsäure hybridisiert.

7. Verfahren nach Anspruch 1, umfassend:
(a) Schmelzen der Probe durch Erhitzen der Probe auf eine erste Temperatur, wobei die erste Temperatur über der Schmelztemperatur (Tm) des Primers liegt, und wobei doppelsträngige Nukleinsäuren in der Probe vorhanden sind, die die Zielnukleinsäure umfassen;
(b) danach Annelieren der Probe durch Verringern der Temperatur auf eine zweite Temperatur, die niedriger als die erste Temperatur ist, um es dem Primer und der Hybridisierungssonde zu ermöglichen, jeweils mit einem einzelsträngigen Abschnitt der Zielnukleinsäure in der Probe zu hybridisieren; und
(c) danach Verlängern des Primers, indem es der Polymerase ermöglicht wird, den mit der Zielnukleinsäure hybridisierten Primer bei einer dritten Temperatur zu erweitern, wodurch das Sondenfragment freigesetzt wird;
(d) optional zumindest einmaliges Wiederholen des Schmelzens, Annelierens und Verlängerns;
(e) Inkontaktbringen der Probe mit einer Oberfläche eines Feststoffträgers, wobei die Oberfläche des Feststoffträgers eine oder mehrere Fängersonden umfasst, die mit zumindest einem Abschnitt der zweiten Region des Sondenfragments hybridisieren;
(f) Ermöglichen, dass die Fängersonden mit zumindest einem Abschnitt des Sondenfragments in der Probe hybridisieren, um einen Sondenfragment/Fängersonde-Komplex bei einer vierten Temperatur zu bilden, die niedriger als die zweite und die dritte Temperatur ist; und
(g) Nachweisen der Markierung auf der Oberfläche des Feststoffträgers.

8. Verfahren nach einem vorstehenden Anspruch, wobei die Fängersonde ein Diskrimierungsverhältnis von 3 oder höher aufweist.

9. Verfahren nach Anspruch 7, wobei die zweite Temperatur und die dritte Temperatur gleich sind.

10. Verfahren nach einem vorstehenden Anspruch, wobei die Polymerase und das Enzym mit Nukleaseaktivität das gleiche Molekül sind, wobei die Polymerase optional ein wärmestabiles Enzym ist.

11. Verfahren nach einem vorstehenden Anspruch, wobei die Oberfläche des Feststoffträgers eine Elektrode umfasst, und wobei die nachweisbare Markierung eine Einheit ist, die Elektronen von der oder zur Elektrode übertragen kann, wobei die nachweisbare Markierung optional eine Ferroceneinheit ist, und/oder wobei die Oberfläche des Feststoffträgers optional Gold umfasst.

12. Verfahren nach einem vorstehenden Anspruch, wobei der Feststoffträger eine Vielzahl von fingerförmig ineinandergreifenden Platten umfasst, die einen Strömungskanal bilden, wobei zumindest einige der Oberflächen der Platten Fängersonden umfassen, und wobei das Inkontaktbringen der Probe mit einer Oberfläche eines Feststoffträgers das Strömen der Probe durch den Strömungskanal umfasst, wobei die Oberflächen der Platten optional Elektroden umfassen, und/oder wobei die Oberflächen von alternierenden Platten optional Fängersonden umfassen.

13. Verfahren nach Anspruch 7, wobei das Schmelzen, das Annelieren und das Verlängern mehrmals in einer Reihe von Zyklen durchgeführt werden, wobei das Nachweisen der Markierung auf der Oberfläche des Feststoffträgers optional nach dem letzten Schmelz-, Annelier- und Verlängerungszyklus erfolgt.

14. Verfahren nach Anspruch 7, wobei die Probe während des Schmelzens, Annelierens und Verlängerns mit der Oberfläche des Feststoffträgers in Kontakt steht, und wobei das Nachweisen mehrmals während des Verfahrens und/oder nach dem letzten Schmelz-, Annelier- und Verlängerungszyklus erfolgt.

15. Kit zum Nachweisen einer Zielnukleinsäure in einer Probe, umfassend:
(a) eine Hybridisierungssonde mit einer ersten Region, die mit zumindest einem Abschnitt der Zielnukleinsäure hybridisiert, und mit einer zweiten Region, die eine nachweisbare Markierung umfasst, wobei die zweite Region nicht mit der Zielnukleinsäure hybridisiert, und wobei ein Enzym mit Nukleaseaktivität die Hybridisierungssonde spalten kann, wenn mit der Zielnukleinsäure hybridisiert, um ein Sondenfragment zu produzieren, das die zweite Region und die nachweisbare Markierung umfasst;
(b) einen Feststoffträger, der eine oder mehrere Fängersonden auf einer Oberfläche davon umfasst, wobei die Fängersonde mit zumindest einem Abschnitt der zweiten Region des Sondenfragments hybridisiert, um einen Sondenfragment/Fängersonde-Komplex zu bilden, wobei die erste Region der Hybridisierungssonde eine Einheit umfasst, die die Bindung der intakten Hybridisierungssonde an die Fängersonde durch sterische Hinderung hemmt, und wobei die Hybridisierungssonde bei der Tm des Sondenfragment/Fängersonde-Komplexes im Wesentlichen einzelsträngig ist;
(c) optional einen Primer, der mit zumindest einem Abschnitt der Zielnukleinsäure hybridisiert; und
(d) optional eine Polymerase, die den hybridisierten Primer in Richtung der hybridisierten Sonde erweitert, und ein Enzym mit Nukleaseaktivität, um die hybridisierte Hybridisierungssonde zu spalten und das Sondenfragment, das die zweite Region der Sonde und die nachweisbare Markierung umfasst, freizusetzen.

16. Kit nach Anspruch 15, wobei die Fängersonde ein Diskrimierungsverhältnis von 3 oder höher aufweist.

17. Kit nach Anspruch 15, wobei die Oberfläche des Feststoffträgers eine Elektrode umfasst, und wobei die nachweisbare Markierung eine Einheit ist, die Elektronen von der oder zur Elektrode übertragen kann, vorzugsweise wobei die nachweisbare Markierung optional eine elektroaktive Ferroceneinheit ist; und/oder wobei die Oberfläche des Feststoffträgers optional Gold umfasst.

18. Kit nach Anspruch 15, wobei die Polymerase und das Enzym mit Nukleaseaktivität das gleiche Molekül sind.

19. Verfahren nach einem der Ansprüche 1 bis 14 oder Kit nach einem der Ansprüche 15 bis 18, wobei die Nukleaseaktivität Exonukleaseaktivität ist.

20. Verfahren nach Anspruch 8, wobei die Fängersonde ein Diskrimierungsverhältnis von 5 oder höher aufweist.

21. Verfahren nach Anspruch 10, wobei die Polymerase eine Taq-Polymerase ist.

22. Kit nach Anspruch 16, wobei die Fängersonde ein Diskrimierungsverhältnis von 5 oder höher aufweist.

## Revendications

1. Procédé de détection d'un acide nucléique cible dans un échantillon, le procédé comprenant les opérations consistant à :
(a) faire incuber l'échantillon avec :
(1) une amorce qui s'hydride à au moins une partie de l'acide nucléique cible ;
(2) une sonde d'hybridation comprenant des première et deuxième régions, la première région s'hybridant à au moins une partie de l'acide nucléique cible et la deuxième région ne s'hybridant pas à l'acide nucléique cible, la deuxième région comprenant un marqueur détectable ; et
(3) une polymérase et une enzyme comprenant une activité nucléase, la polymérase étendant l'amorce hybridée dans la direction de la sonde hybridée et l'activité nucléase de l'enzyme clivant la sonde hybridée, permettant ainsi de libérer un fragment de sonde comprenant la deuxième région et le marqueur détectable ;
(b) amener l'amorce et la sonde d'hybridation à s'hybrider à l'acide nucléique cible dans l'échantillon ;
(c) amener la polymérase à étendre l'amorce hybridée ;
(d) amener l'activité nucléase de l'enzyme à cliver la sonde d'hybridation hybridée, permettant ainsi de libérer le fragment de sonde ;
(e) mettre en contact l'échantillon avec une surface d'un support solide, la surface du support solide comprenant une ou plusieurs sondes de capture dont chacune s'hybride à au moins une partie de la deuxième région du fragment de sonde ;
(f) amener les sondes de capture à s'hybrider au fragment de sonde dans l'échantillon pour former un complexe fragment de sonde/sonde de capture ; et
(g) détecter le marqueur sur la surface du support solide, la sonde d'hybridation étant sensiblement à simple brin à la Tm du complexe fragment de sonde/sonde de capture, et la première région de la sonde d'hybridation comprenant une fraction qui inhibe la liaison de la sonde d'hybridation intacte à la sonde de capture par encombrement stérique.

2. Procédé selon la revendication 1, dans lequel la deuxième région du fragment de sonde se lie à la sonde de capture de telle sorte que la partie de la deuxième région adjacente à la première région dans la sonde d'hybridation intacte est orientée vers la surface de support solide, où, facultativement, une région proximale de la sonde de capture adjacente à la surface de support solide ne s'hybride pas au fragment de sonde et une région distale de la sonde de capture éloignée de la surface de support solide s'hybride à la deuxième région du fragment de sonde.

3. Procédé selon la revendication 2, dans lequel la région proximale de la sonde de capture est plus courte que la première région de la sonde d'hybridation.

4. Procédé selon la revendication 1, dans lequel la sonde de capture et la sonde d'hybridation comprennent chacun des polynucléotides, les polynucléotides comprenant facultativement des désoxyribonucléotides.

5. Procédé selon la revendication 4, dans lequel la région proximale de la sonde de capture a moins de nucléotides que la première région de la sonde d'hybridation intacte.

6. Procédé selon la revendication 1, dans lequel la sonde d'hybridation comprend en outre une troisième région adjacente à la première région et opposée à la deuxième région, la troisième région ne s'hybridant pas à l'acide nucléique cible.

7. Procédé selon la revendication 1, comprenant les opérations consistant à :
(a) faire fondre l'échantillon par chauffage de l'échantillon à une première température, la première température étant au-dessus de la température de fusion (Tm) de l'amorce et des acides nucléiques double brin étant présents dans l'échantillon, lesquels comprennent l'acide nucléique cible ;
(b) par la suite hybrider l'échantillon par réduction de la température à une deuxième température inférieure à la première température pour permettre à l'amorce et à la sonde d'hybridation de s'hybrider chacune à une partie simple brin de l'acide nucléique cible dans l'échantillon ; et
(c) par la suite allonger l'amorce en amenant la polymérase à étendre l'amorce hybridée à l'acide nucléique cible à une troisième température, permettant ainsi de libérer le fragment de sonde ;
(d) facultativement répéter la fusion, l'hybridation et l'allongement au moins une fois ;
(e) mettre en contact l'échantillon avec une surface d'un support solide, la surface du support solide comprenant une ou plusieurs sondes de capture qui s'hybrident à au moins une partie de la deuxième région du fragment de sonde ;
(f) amener les sondes de capture à s'hybrider à au moins une partie du fragment de sonde dans l'échantillon pour former un complexe fragment de sonde/sonde de capture à une quatrième température inférieure aux deuxième et troisième températures ; et
(g) détecter un marqueur sur la surface du support solide.

8. Procédé selon l'une quelconque des revendications précédentes, dans lequel la sonde de capture a un rapport de discrimination de 3 ou plus.

9. Procédé selon la revendication 7, dans lequel la deuxième température et la troisième température sont identiques.

10. Procédé selon l'une quelconque des revendications précédentes, dans lequel la polymérase et l'enzyme comprenant l'activité nucléase sont la même molécule, la polymérase étant facultativement une enzyme thermostable.

11. Procédé selon l'une quelconque des revendications précédentes, dans lequel la surface du support solide comprend une électrode, et dans lequel le marqueur détectable est une fraction qui peut transférer des électrons à ou à partir de l'électrode, le marqueur détectable étant facultativement une fraction ferrocène, et/ou la surface du support solide comprenant facultativement de l'or.

12. Procédé selon l'une quelconque des revendications précédentes, dans lequel le support solide comprend une pluralité de plaques interdigitées formant un canal d'écoulement, au moins une partie des surfaces des plaques comprenant des sondes de capture, et dans lequel la mise en contact de l'échantillon avec une surface d'un support solide comprend l'écoulement de l'échantillon à travers le canal d'écoulement, les surfaces des plaques comprenant facultativement des électrodes, et/ou les surfaces de plaques alternées comprenant facultativement des sondes de capture.

13. Procédé selon la revendication 7, dans lequel la fusion, l'hybridation et l'allongement sont réalisés de multiples fois dans une série de cycles, la détection du marqueur sur la surface du support solide ayant lieu facultativement après le dernier cycle de fusion, d'hybridation et d'allongement.

14. Procédé selon la revendication 7, dans lequel l'échantillon est en contact avec la surface du support solide durant la fusion, l'hybridation et l'allongement, et dans lequel la détection a lieu plusieurs fois durant le procédé et/ou après le dernier cycle de fusion, d'hybridation et d'allongement.

15. Coffret de détection d'un acide nucléique cible dans un échantillon comprenant :
(a) une sonde d'hybridation comprenant une première région qui s'hybride à au moins une partie de l'acide nucléique cible et une deuxième région comprenant un marqueur détectable, la deuxième région ne s'hybridant pas à l'acide nucléique cible, et une enzyme comprenant une activité nucléase pouvant cliver la sonde d'hybridation lorsqu'elle est hybridée à l'acide nucléique cible, permettant ainsi de produire un fragment de sonde comprenant la deuxième région et le marqueur détectable ;
(b) un support solide comprenant une ou plusieurs sondes de capture sur une surface de celui-ci, la sonde de capture s'hybridant à au moins une partie de la deuxième région du fragment de sonde pour former un complexe fragment de sonde/sonde de capture, la première région de la sonde d'hybridation comprenant un fragment qui inhibe la liaison de la sonde d'hybridation intacte à la sonde de capture par encombrement stérique, et la sonde d'hybridation étant sensiblement à simple brin à la Tm du complexe fragment de sonde/sonde de capture ;
(c) facultativement, une amorce qui s'hybride à au moins une partie de l'acide nucléique cible ; et
(d) facultativement, une polymérase qui étend l'amorce hybridée dans la direction de la sonde hybridée et une enzyme comprenant une activité nucléase, permettant ainsi de cliver la sonde d'hybridation hybridée et de libérer le fragment de sonde comprenant la deuxième région de la sonde et le marqueur détectable.

16. Coffret selon la revendication 15, dans lequel la sonde de capture a un rapport de discrimination de 3 ou plus.

17. Coffret selon la revendication 15, dans lequel la surface du support solide comprend une électrode et dans laquelle le marqueur détectable est une fraction qui peut transférer des électrons à ou à partir de l'électrode, où de préférence le marqueur détectable est facultativement une fraction ferrocène électro-active, et/ou la surface du support solide comprenant facultativement de l'or.

18. Coffret selon la revendication 15, dans lequel la polymérase et l'enzyme comprenant l'activité nucléase sont la même molécule.

19. Procédé selon l'une quelconque des revendications 1 à 14, ou coffret selon l'une quelconque des revendications 15 à 18, dans lequel l'activité nucléase est une activité exonucléase.

20. Procédé selon la revendication 8, dans lequel la sonde de capture a un rapport de discrimination de 5 ou plus.

21. Procédé selon la revendication 10, dans lequel la polymérase est une Taq polymérase.

22. Coffret selon la revendication 16, dans lequel la sonde de capture a un rapport de discrimination de 5 ou plus.
